# EUROPEAN PATENT APPLICATION

(11) **EP 4 000 420 A1**
(43) Date of publication of application: **25.05.2022**
(21) Application number: 20840908.6
(22) Date of filing: 03.07.2020
(51) Int. Cl.: A23L 33/00

(54) **EDIBLE COMPOSITION FOR PROMOTING HAIR GROWTH AND DELAYING GRAY HAIR GENERATION**

(30) Priority: 17.07.2019 CN 201910643777
(71) Applicant: Shanghai Lytone Biochemicals, Ltd., Shanghai 200050 (CN)
(72) Inventor: LIU, Huanhuan, Shanghai 200050 (CN); CHANG, Weiting, Shanghai 200050 (CN); CHANG, William T.H., Shanghai 200050 (CN); TSENG, Weiting, Shanghai 200050 (CN); CHEN, Minghui, Shanghai 200050 (CN)
(74) Representative: Dehns
(86) International application number: PCT/CN2020/100100
(87) International publication number: WO 2021/008386

(57) **Abstract**

Disclosed is an edible composition for promoting hair growth and delaying gray hair generation. The composition comprises, in parts by weight, 2-30 parts of nucleoprotein peptide, 1-25 parts of whole coffee cherry extract, 5-60 parts of composite yeast powder, and 10-55 parts of collagen. The edible composition for promoting hair growth and delaying gray hair generation can promote hair growth. Fine hair can change significantly after 1 week of consumption, and fine hair can increase by 2 to 3 times after 8 weeks of consumption. The gray hair rate is significantly reduced after 2 weeks of consumption, and the gray hair rate is reduced by 10% - 40% after 8 weeks of consumption. Same also has the effect of improving skin quality.

## Description

### Cross-Reference to Related Applications

The present application claims priority to Chinese patent application No. 201910643777.2, filed on 17 July 2019, with the title "Edible Composition for Promoting Hair Growth and Delaying Hair Graying", which is hereby incorporated by reference in its entirety.

### Technical Field

The present invention relates to an edible composition for promoting hair growth and delaying hair graying, which pertains to the field of food technology.

### Background

Hair loss and premature hair graying are common clinical hair disorders that have a major negative impact on people's mental and psychological well-being. In recent years, with the increasing competition in society, the pressure of people at work, in studying and in life gradually increases, leading to an elevated incidence of hair loss and premature hair graying year by year, with the age of onset getting younger. According to an urban sample survey, the prevalence of grey hair and hair loss among the middle-aged (aged 35-59) in China is as high as 45.7%. As an appendage of the human skin, the hair not only protects the brain, reducing and avoiding external mechanical and chemical damages and cushioning damage to the head, but also stops or reduces the damage to the scalp and intracranial tissues and organs by UV. In addition, the hair also metabolizes harmful substances in the body such as heavy metals including mercury, arsenic and lead, provides an outlet for secretions from the sebaceous and sweat glands, promotes cellular metabolism, improves blood circulation, and strengthens immunity. In addition, the hair directly affects the appearance of a person and has significant cosmetic values.

It is considered in modern medicine that grey hair is resulted from the diminished functioning of melanocytes in the hair bulb to form melanin, the decreasing or loss of tyrosinase activity and the lack of melanin in the hair shaft. Currently, there is no effective treatment available and most patients disguise it with hair dye. However, in addition to damaging the protective film of the hair, harming the scalp and hair follicles, accelerating keratinization of the hair follicles and causing thinning of the hair, excessive hair dye can also lead to massive hair loss and even major diseases.

### Summary of the Invention

In view of the existing problems in the prior art, an object of the present invention is to provide an edible composition for promoting hair growth and delaying hair graying for people suffering from baldness and premature grey hair. This composition can promote hair growth while delaying hair graying. Where content is indicated, "%" hereinafter indicates "% by weight" unless specified otherwise.

The object of the present invention is achieved by the following technical solutions.

In one aspect, the present invention provides an edible composition comprising in parts by weight:

| | |
|---|---|
| nucleoprotein peptides | 2 to 30 parts; |
| whole coffee fruit extract | 1 to 25 parts; |
| yeast composite powder | 5 to 60 parts; |
| collagen | 10 to 55 parts. |

There has not been any reports in the prior art that any single one of these four components is effective in promoting hair growth, nor are nucleoprotein peptides and whole coffee fruit extract reported to have an activating effect on tyrosinases. Through extensive experiments, the inventors have unexpectedly found that the activation rate of tyrosinases by nucleoprotein peptides and whole coffee fruit extract is 31% and 37%, respectively, and the activation rate can be increased to up to 44% with a combination of both in a proper ratio. Further, the yeast composite powder and collagen provide nutrients for the growth of hair follicles, thereby promoting hair growth. The composition obtained by combining the four components in the above-mentioned ratio according to the present invention promotes hair growth while delaying the appearance of grey hair.

In the present invention, the nucleoprotein peptides are a mixture comprising nucleic acids (e.g., DNA and RNA and/or the salt forms thereof) and proteins (e.g., basic proteins such as histones and protamine) and peptides thereof that are extracted from the nuclei of plants, animals or microorganisms. Whole coffee fruit extract is a mixture of extracts from whole coffee fruits. The raw materials used in the present invention, such as nucleoprotein peptides, whole coffee fruit extract, yeast composite powder and collagen, are commercially available (e.g. nucleoprotein peptides from Maruha Nichiro) or can be prepared with techniques in the art. For example, nucleoprotein peptides can be made from salmon sperm by enzymatic digestion, concentration and drying; whole coffee fruit extract can be made from coffee fruit after extraction in an aqueous ethanol solution, concentration and drying. The raw materials should meet the quality requirements of the relevant industry standards.

In some embodiments, the content of sodium deoxyribonucleic acid (DNA-Na) in the nucleoprotein peptides in the edible composition of the present invention is preferably 86% or more.

In some embodiments, in the edible composition of the present invention, the content of chlorogenic acid in the whole coffee fruit extract is 30% or more, for example 40% or more, 50% or more, 60% or more, 70% or more, 80% or more, or even 90% or more. The amount of whole coffee fruit extract in the edible composition of the present invention can be properly adjusted according to the level of the chlorogenic acid content in the whole coffee fruit extract. In further embodiments, the whole coffee fruit extract comprises 20% to 50% total chlorogenic acids and 0.5% to 5% proanthocyanidin, wherein the total chlorogenic acids include neochlorogenic acid and the neochlorogenic acid is comprised in the whole coffee fruit extract at from 1% to 20%.

In some embodiments, in the edible composition of the present invention, pantothenic acid is comprised in the yeast composite powder at 1.1% or more. The amount of the yeast composite powder in the edible composition of the present invention can be adjusted appropriately according to the level of the pantothenic acid content in the yeast composite powder.

Preferably, in the above edible composition, the content of the nucleoprotein peptides is from 2% to 30%, based on a total mass of the composition of 100%.

Preferably, in the above edible composition, the content of the whole coffee fruit extract is from 1% to 25%, based on a total mass of the composition of 100%.

Preferably, in the above edible composition, the content of the yeast composite powder is from 5% to 60%, based on a total mass of the composition of 100%.

Preferably, in the above edible composition, the content of collagen is from 10% to 55%, based on a total mass of the composition of 100%.

Preferably, in the above edible composition, the composition consists of nucleoprotein peptides, whole coffee fruit extract, yeast composite powder, and collagen.

In a further aspect, the present invention also provides the use of the edible composition in promoting hair growth. Particularly, provided is the use of the edible composition in the manufacture of a food having an effect in promoting hair growth.

In yet a further aspect, the present invention also provides the use of the edible composition in delaying hair graying. Particularly, provided is the use of the edible composition in the manufacture of a food having an effect in delaying hair graying.

In still a further aspect, the present invention also provides the use of the edible composition in improving the skin tone. Particularly, provided is the use of the edible composition in the manufacture of a food having an effect in skin improvement.

According to embodiments of the present invention, the food for the aforementioned use may optionally comprises some excipients in addition to an effective amount of the edible composition according to the invention. The excipients may be one or a combination of, but not limited to, excipients commonly used in the food industry, such as sorbitol, magnesium stearate and maltodextrin.

According to embodiments of the present invention, the food for the aforementioned use may be in the form of tablets, capsules, powder or granules, but not limited thereto. The preparation of the tablets, capsules, powder or granules may be carried out in accordance with the prior art.

The edible composition provided by the present invention is able to promote hair growth. After it is consumed for one week, there is a significant change in fine hair, with the fine hairs increased by 2 to 3 times after 8 weeks of consumption. Further, the percentage of grey hair is significant reduced after 2 weeks of consumption, with a 10% to 40% reduction in the percentage of grey hair after 8 weeks of consumption. Subjects show improved skin firmness after 3 weeks of consumption, with a remarkably younger-looking skin after 5 weeks of consumption.

### Brief Description of the Drawings

Fig. 1 shows a comparison in the activation of tyrosine kinase by various components in the Examples.
Fig. 2 shows a comparison of the changes in hair counts before and after the consumption of the composition of the present invention in the Examples.
Fig. 3 shows a comparison of the changes in the percentage of grey hair and the percentage of fine hair after the consumption of the composition of the present invention in the Examples.
Fig. 4 shows a comparison of the changes in hair follicles before and after the consumption of the composition of the present invention in the Examples.
Fig. 5 shows a comparison of the conditions of grey hair before and after the consumption of the composition of the present invention in the Examples.
Fig. 6 shows a comparison of the changes in skin firmness before and after the consumption of the composition of the present invention in the Examples.
Fig. 7 shows a comparison of the changes in skin age after consumption of the composition of the present invention in the Examples.
Fig. 8 shows a comparison of the conditions of grey hair, the percentages of fine hair and the conditions of dark hair before and after the consumption of another composition of the present invention in the Examples.
Fig. 9 shows a comparison of the changes in skin firmness before and after the consumption of another composition of the present invention in the Examples.

### Detailed Description of the Invention

In order to provide a better understanding of the technical features, objects and advantageous effects of the present invention, detailed description of the technical solutions of the present invention is provided herein below, which is not to be construed as limiting the implementable scope of the present invention.

### Example 1

This example provides a composition for promoting hair growth and delaying hair graying, wherein the composition comprises in parts by weight:

| | |
|---|---|
| nucleoprotein peptides (DNA-Na content >86%) | 5 parts; |
| whole coffee fruit extract (chlorogenic acid content >30%) | 3 parts; |
| yeast composite powder (pantothenic acid >1.1%) | 14 parts; |
| collagen | 20 parts; |
| sorbitol | 57.8 parts; |
| magnesium stearate | 0.2 parts. |

The composition was prepared as follows: nucleoprotein peptides (purchased from Maruha Nichiro Corp.), whole coffee fruit extract (obtained by extracting coffee fruits in an aqueous ethanol solution, concentrating and drying), yeast composite powder, collagen, sorbitol and magnesium stearate were proportionally weighed and uniformly mixed, and then pressed into tablets to obtain the composition for promoting hair growth and delaying hair graying.

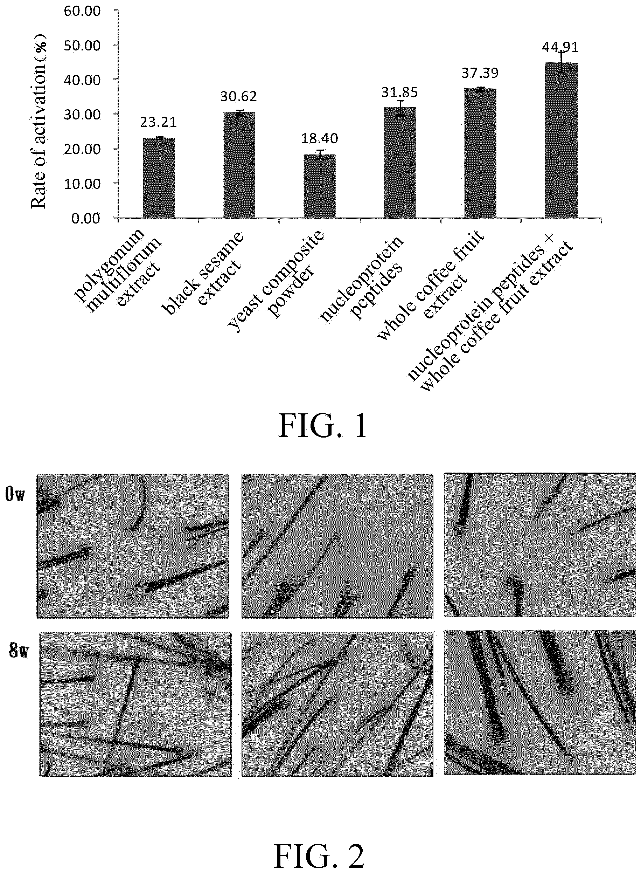

In this example, comparative experiments were carried out for the activation rate of tyrosinase by the raw material whole coffee fruit extract, with *polygonum multiflorum* extract, black sesame extract, yeast composite powder, and nucleoprotein peptides used in the comparative examples, respectively. The results of the experiments are shown in Fig. 1. As can be seen from Fig. 1, the activation rate of tyrosinase by the whole coffee fruit extract reaches 37.39%, and the activation rate of tyrosinase by the whole coffee fruit extract in combination with nucleoprotein peptides reaches 44.91%, which are higher than the other comparative examples.

### Example 2

This example provides a composition for promoting hair growth and delaying hair graying, wherein the composition comprised, in parts by weight:

| | |
|---|---|
| nucleoprotein peptides (DNA-Na content >86%) | 8 parts; |
| whole coffee fruit extract (chlorogenic acid content >30%) | 9 parts; |
| yeast composite powder (>1.1% pantothenic acid) | 22 parts; |
| collagen | 23 parts; |
| maltodextrin | 38 parts |

The composition was prepared as follows: nucleoprotein peptides (purchased from Maruha Nichiro Corp.), whole coffee fruit extract (obtained by extracting coffee fruits in an aqueous ethanol solution, concentrating and drying), yeast composite powder, collagen and maltodextrin were proportionally weighed and uniformly mixed, and formulated into capsules of the composition for promoting hair growth and delay hair graying.

### Application Example 1

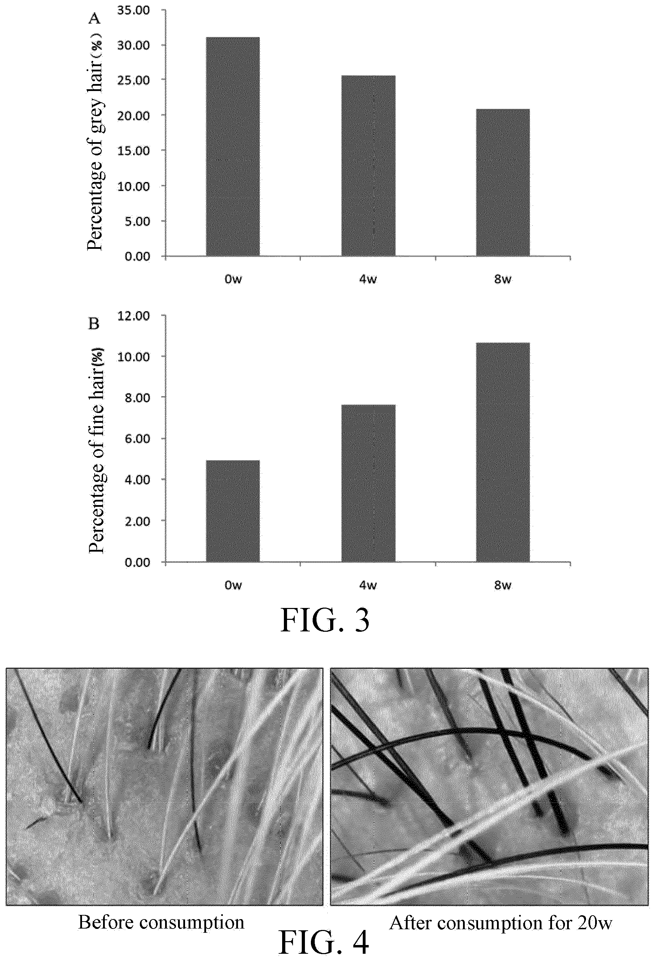

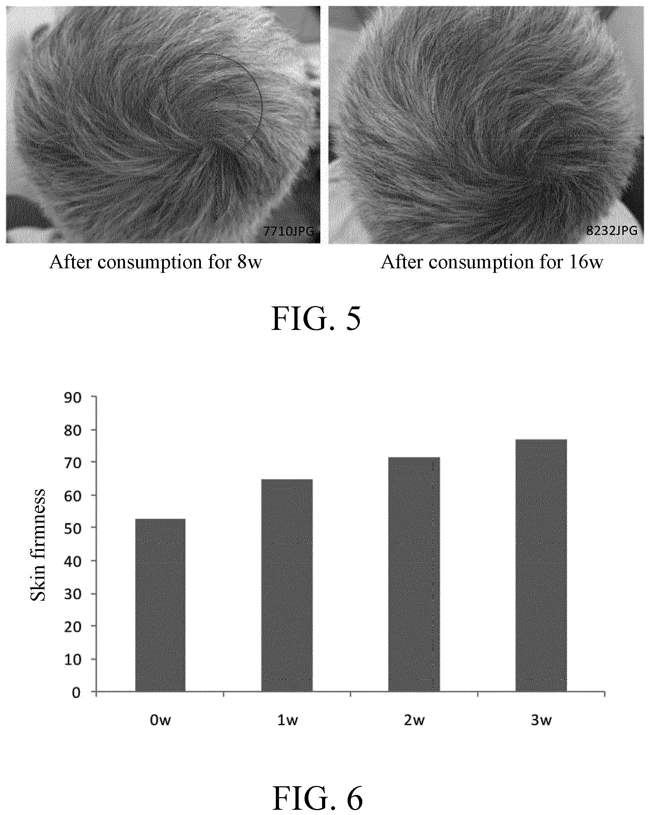

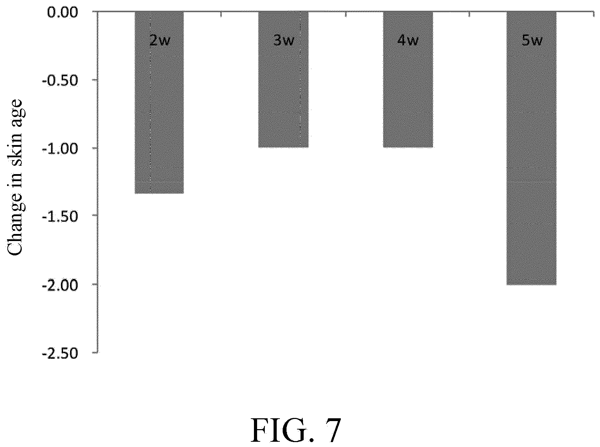

The composition of Example 1 was used in the treatment of hair loss and premature hair graying. The results of the experiment are shown in Figs. 2 and 3. As can be seen from panel A in Fig. 3, the percentage of grey hair was significantly reduced after 2 weeks of consumption and decreased by 10% to 40% after 8 weeks of consumption. As can be seen from panel B in Fig. 3, the fine hair were significantly changed after 1 week of consumption and increased by 2 to 3 times after 8 weeks of consumption. With the period of consumption time extended, the percentage of grey hair was remarkably reduced, as shown in Figs. 4 and 5. As such, the composition of the present invention can promote hair growth and delay hair graying at the same time. In addition, the skin firmness of the subjects was improved after 3 weeks of consumption (see Fig. 6), and the skin age also decreased after 5 weeks of consumption, as shown in Fig. 7.

### Application Example 2

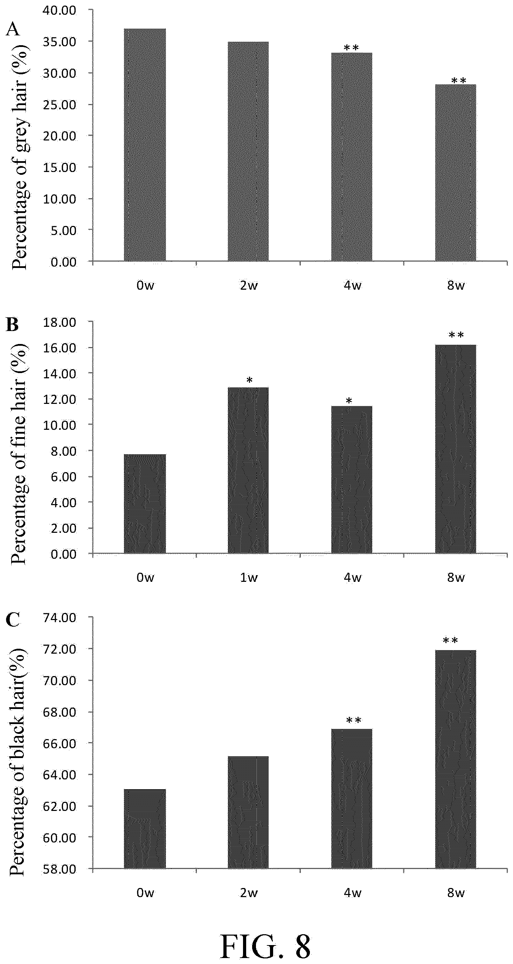

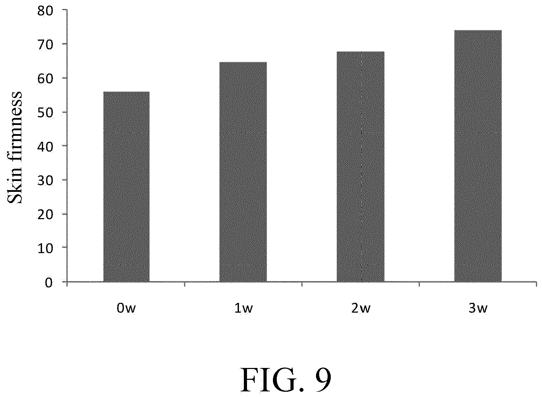

1.6 g of the capsules of the composition prepared according to Example 2 was taken daily by subjects, and the condition of the hair follicles at fixed locations on the subjects' head was monitored weekly with a hair follicle instrument, photographed and the number of hairs were counted; at the same time, the condition of the skin at fixed locations on the subjects' face was also monitored with a skin detector. The results of the experiments are shown in Figs. 8 and 9. As can be seen in Fig. 8, there was a significant decrease in the percentage of grey hair (Fig. 8, panel A, P < 0.01) and a significant increase in the percentage of dark hair (Fig. 8, panel B, P < 0.01) after 8 weeks of consumption, as well as a significant increase in the percentage of fine hair after 1 week of consumption (Fig. 8, panel C, P < 0.05). As such, the composition of the present invention can promote hair growth, and delay hair graying at the same time. In addition, after 3 weeks of consumption, the skin firmness of the subjects was improved, as shown in Fig. 9.

## Claims

1. An edible composition comprising in parts by weight:
| | |
|---|---|
| nucleoprotein peptides | 2 to 30 parts; |
| whole coffee fruit extract | 1 to 25 parts; |
| yeast composite powder | 5 to 60 parts; |
| collagen | 10 to 55 parts. |

2. The edible composition according to claim 1, wherein the content of the nucleoprotein peptides is from 2% to 30%, based on a total mass of the composition of 100%.

3. The edible composition according to claim 1, wherein the content of the whole coffee fruit extract is from 1% to 25%, based on a total mass of the composition of 100%.

4. The edible composition according to claim 1, wherein the content of the yeast composite powder is from 5% to 60%, based on a total mass of the composition of 100%.

5. The edible composition according to claim 1, wherein the content of collagen is from 10% to 55%, based on a total mass of the composition of 100%.

6. The edible composition according to claim 1, wherein the composition consists of nucleoprotein peptides, whole coffee fruit extract, yeast composite powder, and collagen.

7. The edible composition according to claim 1, wherein the nucleoprotein peptides are nucleoprotein peptide compositions with a sodium deoxyribonucleic acid content of 86% or more.

8. The edible composition according to claim 1, wherein the whole coffee fruit extract comprises 30% or more of chlorogenic acid.

9. The edible composition according to claim 1, the whole coffee fruit extract comprises 20% to 50% of total chlorogenic acids and 0.5% to 5% of proanthocyanidin.

10. The edible composition according to claim 9, wherein the total chlorogenic acids include neochlorogenic acid, and the neochlorogenic acid is present in the whole coffee fruit extract in a percentage of 1% to 20%.

11. Use of the edible composition according to any one of claims 1 to 10 in the manufacture of a food having an effect in promoting hair growth.

12. Use of the edible composition according to any one of claims 1 to 10 in the manufacture of a food having an effect in delaying hair graying.

13. Use of the edible composition according to any one of claims 1 to 10 in the manufacture of a food having an effect in skin improvement.

14. The use according to any one of claims 11 to 13, wherein the food comprises an effective amount of the edible composition according to any one of claims 1 to 6, optionally further comprising an excipient.

15. The use according to claim 14, wherein the food is in the form of tablets, capsules, powder or granules.
